(19) Europäisches Patentamt
European Patent Office
Office européen des brevets

(11) EP 0 856 314 A1

(12) # EUROPEAN PATENT APPLICATION
published in accordance with Art. 158(3) EPC

(43) Date of publication:
**05.08.1998 Bulletin 1998/32**

(21) Application number: **96908373.2**

(22) Date of filing: **08.04.1996**

(51) Int. Cl.$^6$: **A61K 35/14**, A61K 39/00

(86) International application number:
**PCT/JP96/00959**

(87) International publication number:
**WO 96/31224 (10.10.1996 Gazette 1996/45)**

(84) Designated Contracting States:
**AT BE CH DE DK ES FI FR GB GR IE IT LI LU MC NL PT SE**

(30) Priority: **07.04.1995 JP 82831/95**
**05.04.1996 JP 83766/96**

(71) Applicant:
**Chugai Seiyaku Kabushiki Kaisha**
**Tokyo 115 (JP)**

(72) Inventor:
**NAGAMUTA, Masahiro,Chugai Seiyaku Kabushiki Kaisha**
**Kamiina-gun, Nagano 396-46 (JP)**

(74) Representative:
**Woods, Geoffrey Corlett**
**J.A. KEMP & CO.**
**14 South Square**
**Gray's Inn**
**London WC1R 5LX (GB)**

(54) **IMMUNOSUPPRESSANT**

(57) The present invention relates to an immunosuppressant comprising antigen-treated B lymphocytes obtained by contact-treating B lymphocytes with an antigen, a liposome encapsulating or attaching to itself an antigen, or a conjugate of an antigen and a substance which binds to B lymphocytes, or a mixture of at least two of these substances; a method for creating an immunosuppressed animal comprising transferring the immunosuppressant to an animal; an immunosuppressed animal created by the above method; a method for treating or preventing diseases comprising administering the immunosuppressant to a human; and the liposome and the conjugate described above.



Printed by Xerox (UK) Business Services
2.16.3/3.4

## Description

### Technical Field

The present invention relates to an immunosuppressant, an immunosuppressed animal into which the immunosuppressant has been transferred, a method for creating the immunosuppressed animal, and a method for treating or preventing diseases using the immunosuppressant.

### Background Art

Recently, immunosuppressants have been used widely in clinical scenes of organ transplantation.

However, these formulations act non-specifically on the immune system and thus involve a danger of causing side effects including not only exogenous infections but also development of endogenous diseases such as induction of malignant tumors. Therefore, target diseases in which these formulations may be used are extremely limited. Under circumstances, an agent which induces a suppressive effect specific to a target antigen is needed.

As a method of inducing an antigen-specific immunosuppression, there has been reported a method in which a spleen-derived cell and an antigen are coupled through 1-ethyl-3-(3-dimethylaminopropyl)carbodiimide (ECDI) and intravenously transplanted to thereby reduce the reactivity of T lymphocytes (M. K. Jenkins, J. Exp. Med., vol. 165, 302 (1987)). In this method, however, development of autoimmune diseases is apprehended because new antigenicity will be revealed in self or the exogenous substance due to the denaturing of autoantigen concurring at the time of ECDI treatment or due to the chemical bonding of ECDI. Also, induction of unexpected bioreactions by the remaining ECDI is anticipated. In view of this, there is a possibility that side effects may be caused. Also, the cell which is responsible for inducing immunosuppression is not elucidated. Furthermore, the influence upon the antibody production is not examined (M.K. Jenkins et al., J. Exp. Med., vol. 165, 302-319 (1987)).

On the other hand, it has been reported recently that B lymphocytes may be the cell which is responsible for inducing an immunosuppressive action (Hori, S.S., J. Immunol. vol. 143, 1447 (1989)). To date, B lymphocytes have been believed to rather activate T lymphocytes to thereby activate the immune system by taking antigens into cells, processing them into fragments, and presenting those fragments on the cell surface together with major histocompatibility complex (MHC) antigens. However, it has been reported that by administering an antibody prepared from an animal of different species having a binding property to B lymphocyte surface antigens, antibody producing ability against the animal-derived antibody is suppressed, and thus it has been proved indirectly that B lymphocytes also have an antibody production suppression action (E. E. Eyron, J. Exp. Med. vol. 175, 131 (1992); S. C. Morris, J. Immunol. vol. 152, 3768 (1994); S. C. Morris, J. Immunol. vol. 152, 3777 (1994)). However, it should be noted here that an antigenic substance which is able to induce immunosuppression is limited to such an antibody derived from an animal of different species that recognizes IgD, CD23 or CR1. Furthermore, it has not been directly proved in this system that B cells are actually responsible for inducing immunosuppression.

Fuchs, E. J. et al. (Science, vol. 258, 1156 (1992)) have directly proved that B lymphocytes are the cells which are responsible for inducing immunosuppression. Briefly, they have reported that by transferring a specific antigen expressing mouse-derived B lymphocytes into a mouse not expressing the antigen, induction of cytotoxic T cells which recognize the above antigen is suppressed. In this system, however, it is difficult to induce an antibody production suppression action against any antigen other than the one actually used. It is understood that, in this system, suppression can be induced against only partial antigens present on the surface of B lymphocytes. Also, there are many unknown points on antibody production ability against a specific antigen.

Accordingly, it has been completely unknown that B lymphocytes are able to induce an immunosuppressive action against any antigen.

### Disclosure of the Invention

It is an object of the invention to provide an immunosuppressant comprising B lymphocytes which are able to suppress an immune reaction with an antigen.

Toward the solution of the above assignment, the present inventors have made intensive and extensive researches on agents which exhibit an antigen-specific immunosuppressive action. As a result, the inventors have found that those B lymphocytes which have been cultured with an antigen or treated so that an antigen is taken into the cell (hereinafter referred to as "contact treatment") reveal an excellent suppressive action against antigen-specific T cell reaction and antibody production when transferred into a human or an animal. Thus, the present invention has been achieved.

The present invention relates to an immunosuppressant comprising those cells which are obtained by contact-treating B lymphocytes with an antigen (hereinafter referred to as "antigen-treated B lymphocytes").

Further, the present invention relates to an immunosuppressant comprising antigen-treated B lymphocytes which are obtained by contact-treating B lymphocytes with an antigen, a liposome encapsulating or attaching to itself an antigen (hereinafter sometimes referred to as an "antigen-containing liposome") or a conjugate composed of an antigen and a substance which binds to B lymphocytes, or a mixture of two or more of these substances.

The immunosuppressant described above can be used to suppress the immunity of an animal in such a manner that B lymphocytes are collected from the animal body; an immunosuppressive action is induced in those cells; then, the resultant B lymphocytes are transferred to the animal body.

In the present invention, the term "contact treatment" means such a treatment as culturing in an appropriate medium B lymphocytes with an antigen or a conjugate composed of an antigen and a substance which binds to B lymphocytes (hereinafter sometimes referred to as a "conjugate of antigen/substance binding to B lymphocytes"), or subjecting an antigen-containing liposome and B lymphocytes to cell fusion treatment, or injecting an antigen into B lymphocytes by microinjection, etc.

As the antigen, cell- or tissue-derived antigens such as histocompatibility antigens, causative antigens of allergies, or causative antigens of autoimmune diseases (food-derived antigens, drugs or some substances present therein expected to exhibit antigenicity, or artificial organ related substances) may be enumerated, for example.

As the substance which binds to B lymphocytes, an antibody, a part thereof, or a crosslinking agent may be enumerated, for example.

As the conjugate of antigen/substance binding to B lymphocytes, a conjugate obtained by expressing with genetic engineering techniques a gene comprising a gene encoding an antigen and a gene encoding a substance which binds to B lymphocytes may be given, in addition to a compound of the above-mentioned antigen and the above-mentioned substance which binds to B lymphocytes.

The present invention further relates to a method of creating an immunosuppressed animal by transferring the immunosuppressant to an animal.

The present invention further relates to an immunosuppressed animal created by the method described above. As the animal, experimental animals may be given.

The present invention further relates to a method of treating or preventing diseases comprising administering the immunosuppressant to a human. The immunosuppressant of the invention may be used for graft rejection, allergy, autoimmune disease, graft-versus-host disease or the like.

Further, the present invention relates to an antigen-containing liposome which encapsulates or attaches to itself an antigen, or a conjugate composed of an antigen and a substance which binds to B lymphocytes. Such a liposome or conjugate may be alone or may be mixed together to form a composition.

In the present invention, a conjugate obtained by expressing a gene comprising a gene encoding an antigen and a gene encoding a substance which binds to B lymphocytes is also included as one of the conjugate composed of an antigen and a substance which binds to B lymphocytes.

The antigen-containing liposome, the conjugate of antigen/substance binding to B lymphocytes, and the expression product of a gene comprising a gene encoding an antigen and a gene encoding a substance which binds to B lymphocytes as described above can be used as an antigen for preparing the antigen-treated B lymphocytes of the invention ("an antigen in a broad sense" to be described later).

Further, the present invention relates to an antigen which is able to induce an immunosuppressive action in B lymphocytes by contacting with B lymphocytes. As the B lymphocytes, those obtained from an animal body may be given.

Hereinbelow, the present invention will be described in detail.

The immunosuppressant of the present invention can be prepared by contact-treating B lymphocytes with an antigen *per se*, an antigen-containing liposome, a conjugate of antigen/substance binding to B lymphocytes, or a mixture of two or more of these substances.

(1) Isolation of B Lymphocytes

B lymphocytes (hereinafter sometimes referred to as "B cells") are present in primary organs of the lymphatic system (such as the bone marrow), secondary organs of the lymphatic system such as lymph nodes (including the blood, the spleen or the mesentery), or other various organs and tissues of a human or animal. B cells are isolated from these tissues by the method described below.

Briefly, blood collected from a human or animal, or a spleen or mesentery obtained by an abdominal operation of an animal is suspended in an appropriate cell culture solution, e.g. MEM, or RPMI-1640 medium containing fetal calf serum (FCS) or bovine serum albumin. With respect to individual lymph nodes including mesenteric lymph nodes, they are cut into small pieces with scissors. The spleen is left in the state as it has been removed. With respect to various organs and tissues, they are treated with an enzyme such as collagenase or DNase or a chelating agent such as EDTA. Then, each of these substances are placed on a metal mesh, and a slight pressure is applied downward to thereby release the lymphocytes from the inside of tissues to the outside. In order to improve the survival ratio Of cells, it is preferable to conduct the enzyme treatment under conditions as moderate as possible. Those cells which passed through the metal mesh are subjected to light pipetting. Thereafter, the cells are centrifuged at about 100xG to thereby obtain a supernatant. By removing tissue fragments and associated cells precipitating, lymphocytes in the form of a single cell are obtained.

Subsequently, from the above-mentioned suspension of blood or single cell, the lymphocyte fraction is obtained by a conventional lymphocyte separation method (e.g. Ficoll density gradient centrifugation). However, this lymphocyte separation operation is not necessarily required. From the lymphocyte containing suspension obtained, B cells are obtained by a conventional B cell separation method through negative selection, positive selection or a combination thereof.

For example, in negative selection, an anti-T cell antibody such as anti-Thy-1 antibody, anti-CD4+CD8 antibody, etc. is added to the lymphocyte containing suspension and allowed to bind to T cells at a low temperature. Then, a complement of a low cytotoxicity is added thereto and reacted at 37°C to thereby remove T cells. Usually, cultivation after the addition of a complement can be performed in 30-40 minutes. It is desirable to perform this cultivation in the necessary minimum time. Alternatively, an anti-T cell antibody bound to magnetic beads is added to the lymphocyte containing suspension and reacted. Thereafter, T cells are removed with a magnet. Alternatively, a fluorescence-labeled anti-T cell antibody is added to the lymphocyte containing suspension and reacted. Thereafter, T cells are removed with a flow cytometer.

On the other hand, in positive selection, an anti-B cell antibody bound to magnetic beads or labeled with fluorescence is added to the lymphocyte containing suspension and then B cells are separated with a magnet or a flow cytometer, respectively, as described above.

In any of these techniques, it is preferable to perform a low temperature treatment to keep cells in better conditions, except for those cases where such treatment is necessary. It is expected that a small number of dendritic cells are present in the thus obtained remaining suspension (B cell fraction). Since dendritic cells have an action to enhance antibody production against an antigen, the presence of these dendritic cells is considered to influence greatly on immunosuppressive action. Thus, it is desirable to remove these dendritic cells as much as possible. For example, dendritic cells and macrophages can be removed by passing the B cell-containing suspension through a Sephadex G-10 column twice. Since B cells are not adsorbed on the column but flow out, the non-adhesive cells eluted are obtained as B cells.

As the animal, experimental animals such as mouse, rat, guinea pig, hamster, rabbit, cat, dog, pig, monkey, and the like may be enumerated.

(2) An Antigen

1) In the present invention, an "antigen" refers to any antigen which may be the target of immunosuppression regardless of the presence or absence of the activity causing immune response in an organism (antigenicity). Preferably, an antigen is used which is able to induce an immunosuppressive action in B cells by directly contacting with B cells. Specifically, cell- or tissue-derived antigens such as histocompatibility antigens, causative antigens of allergies, or causative antigens of autoimmune diseases (food-derived antigens, drugs or some substances present therein expected to exhibit antigenicity, or artificial organ related substances, or denatured substances thereof (e.g., thermally denatured substance); hereinafter "causative antigen" will be sometimes referred to as "causative substance") may be enumerated, for example.

Cell- or tissue-derived antigens mean all of the products of alleles which the host does not have. In particular, major histocompatibility antigen (MHC antigen) and non-major histocompatibility antigen are main targets for immunosuppression. MHC antigen is an antigenic system which fulfills functions involved in immune reactions of an organism including graft rejection reaction. In this antigenic system, HLA antigen, H-2 antigen, RT1 antigen, Hm-1 antigen, GPLA antigen, RLA antigen, DLA antigen, FLA antigen, SLA antigen, CyLA antigen, RhLA antigen and the like are included. Each of them represents the MHC antigen of human, mouse, rat, Sicilian hamster, guinea pig, rabbit, dog, cat, pig, crab-eating macaque and rhesus monkey, respectively.

As causative substances of allergies, environmental/pollen antigens, fungal antigens, food antigens, artificial antigens and the like are included. Specific examples of environmental/pollen antigens include mites, hose dust, pollen of Japanese cedar and ragweed. Specific examples of fungal antigens include *Candida*, *Alternaria*, *Aspergillus*, *Cladosporium*, and *Penicillium*. Specific examples of food antigens include egg white, cow's milk, soy bean, wheat flour, buckwheat flour, mackerel, sardine, horse mackerel, shrimp, crab, pork, beef and chicken. Specific examples of artificial antigens include drugs and artificial organs. As causative substances of autoimmune diseases, disease-causing antigens corresponding to autoantibodies may be enumerated as shown in Table 1 below.

Table 1.

| Disease | Corresponding Antigen to Autoantibody |
|---|---|
| 1. Hashimoto's thyroiditis<br>2. Primary myxedema | Thyroglobulin, microsome, follicular epithelial cell (thyroid peroxidase), 2nd colloid antigen |
| 3. Thyrotoxicosis, Basedow's disease, Graves' disease | Follicular epithelial surface TSH receptor, microsome |
| 4. Idiopathic hypoparathyroidism (partial) | Chief cell of parathyroid |
| 5. Pernicious anemia | Intrinsic factor-$VB_{12}$ binding site and non-binding site, gastric wall cells |
| 6. Ulcerative colitis | Large intestine epithelial lipopoly-saccharide, lymphocytes |
| 7. Autoimmune atrophic gastritis | Gastric wall cells |
| 8. Idiopathic Addison's disease | Steroid producing cells (adrenal, ovary) |
| 9. Male infertility (partial) | Spermatozoon |
| 10. Autoimmune aspermatogenic orchitis | Spermatozoon |
| 11. Autoimmune oophoritis | Zona pellucida |
| 12. Goodpasture's syndrome | Basement membrane (renal glomerulus, alveolus) |
| 13. Rapidly progressive glomerulonephritis (RPGN) (partial) | Renal glomerular basement membrane |
| 14. Tubulo-interstitial nephritis | Renal tubular basement membrane |
| 15. Membranous glomerulonephritis (partial) | Brush border antigen of proximal tubular epithelium |
| 16. Membranoproliferative glomerulo-nephritis type II | C3 converting enzyme |
| 17. Myasthenia gravis | Acetylcholine receptor at myoneural junction, skeltal muscle stripe |
| 18. Polymyositis, dermatomyositis | Myosin, myoglobin, IgG, ssDNA, nuclear substances (JO-1; histidyl tRNA synthetase, PM-1, Mi) |
| 19. Pemphigus vulgaris | Prickle cell membrane of dermal squamous cell |
| 20. Bullous pemphigoid | Basement membrane-like substance at epidermis-dermis junction |

| | |
|---|---|
| 21. Sympathetic ophthalmia | Uvea, pigmented layer of retina |
| 22. Vogt-Koyanagi-Harada syndrome | Uvea pigment, melanin, ganglioside |
| 23. Lens-induced uveitis | Lens $\alpha$-crystallin |
| 24. Multiple sclerosis | Myelin basic protein, galactocerebroside, ganglioside |
| 25. Autoimmune hemolytic anemia | Erythrocytes |
| 26. Cold agglutinin disease | Erythrocytes (I antigen) |
| 27. Paroxysmal cold hemoglobinuria | Erythrocytes (P antigen) |
| 28. Idiopathic thrombocytopenic purpura | Platelets |
| 29. Postmyocardial syndrome | Cardiac muscle |
| 30. Rheumatic fever | Common antigen of cardiac muscle and A group hemolytic streptococcus |
| 31. Lupoid hepatitis | Histone, other nuclear substances, smooth muscle, microsome |
| 32. Primary biliary cirrhosis | Mitochondria, smooth muscle, cholangiole epithelium, nuclear substances |
| 33. Insulin-dependent diabetes mellitus | Islet cells |
| 34. Insulin-resistant diabetes | Insulin receptor |
| 35. Behcet's disease | Tunica mucosa oris, arterial wall, myelin basic protein, galactocerebroside, asialo-ganglioside |
| 36. Sjogren syndrome | Nuclear substances (SS-A, SS-B), IgG, epithelium of exocrine gland conduit |
| 37. Systemic lupus erythematosus | Nuclear substances (DNA, RNA, nucleo-protein), cells (erythrocyte, lymphocyte, neutrophil, platelet), IgG and so forth |
| 38. Rheumatoid arthritis | IgG, nuclear substances (RANA, ssDNA), lymphocytes |
| 39. Juvenile rheumatoid arthritis (partial) | IgG, nuclear substances, T cells |
| 40. Systemic scleroderma | Nuclear substances (RNA polymerase 1 of nucleolus, ssDNA, Scl-70, topoisomerase 1) |
| 41. CREST syndrome | Nuclear substance (centronuclear muscle) |
| 42. Mixed connective tissue disease | Nuclear substance (U1-RNP) |

The "antigen" is as described above (meaning an antigen in a narrow sense), but a liposome to which the above antigen has been attached or a liposome encapsulating the above antigen or a conjugate of the above antigen and a substance which binds to B lymphocytes comprises the antigen and thus means an antigen in a broad sense. Accordingly, unless otherwise indicated, hereinafter the "antigen" includes an antigen *per se*, an antigen-containing liposome, a conjugate of antigen/substance binding to B lymphocytes, and a mixture of two or more of these substances.

Accordingly, in the present invention, the above-described antigen, antigen-containing liposome or conjugate of antigen/substance binding to B lymphocytes is not only used independently but also used in a mixture of at least two of these substances.

2) In the present invention, the antigen-containing liposome described above may be used.

Generally, a liposome is a kind of artificial lipid membrane. When most phospholipid or glyceroglycolipid is suspended in water at least 50% or more in weight at a temperature above the gel-liquid crystal phase transition temperature inherent in the lipid, closed vesicles of lipid bilayer are automatically formed. A liposome is a closed vesicle comprising a material(s) biologically degradable. Since it is possible to allow the internal aqueous layer or lipid bilayer to retain various substances, a number of attempts have been made to use liposomes as microcapsules.

In the present invention, a liposome is utilized to prepare the antigen-containing liposome as described above. By contact treatment of B cells with the prepared antigen-containing liposome, the antigen-treated B lymphocytes of the invention can be obtained.

In the present invention, the encapsulation of an antigen in a liposome or the attaching of an antigen to a liposome may be performed using conventional techniques. For example, the Ca-EDTA method may be used. A method for contacting the antigen attached to or encapsulated in a liposome with B cells will be described later.

3) Further, in the present invention, a conjugate of antigen/substance binding to B cells may be used in the preparation of the antigen-treated B cells of the invention.

As the conjugate of antigen/substance binding to B cells, for example, a product obtained by binding to the above-mentioned antigen a substance which binds to B cells; or a product obtained by expressing a gene comprising a gene encoding the antigen and a gene encoding a substance which binds to B cells (a product obtained by expressing a gene using genetic engineering techniques) may be enumerated.

As the substance which binds to B cells, for example, anti-IgM antibody, anti-IgD antibody, anti-CD23 antibody, anti-CR1 antibody or a part of these antibodies (Fab, Fc, etc.) or a crosslinking agent may be enumerated. A crosslinking agent useful in the invention is not particularly limited as long as it can bind to B cells at the one end and can bind to an antigen at the other end. For example, bifunctional compounds such as succinimidyl 4-(N-maleimidemethyl) cyclohexane-1-carboxylate (SMCC), sulfosuccinimidyl 4-(N-maleimidemethyl)cyclohexane-1-carboxylate (Sulfo-SMCC), N-succinimidyl 3-(2-piridyldithio)propionate (SPDP) and the like may be enumerated. Specific examples of the conjugate of antigen/substance binding to B cells include a conjugate of an antigen and anti-IgM antibody, a conjugate of an antigen and anti-IgD antibody, a conjugate of an antigen and SMCC, and a conjugate of an antigen and SPDP. Thus, one antigen selected from the group of antigens described above and one substance selected from the group of substances which bind to B cells described above may be combined freely.

As a method for binding the antigen to the substance which binds to B cells, a well known method may be used. For example, an SH group is introduced into the above antigen or the substance which binds to B cells using an SH group introducing agent such as S-acetylmercaptosuccinic anhydride, and subsequently the antigen and the substance are bound by using one of the crosslinking agents described above (e.g., SMCC) or the like.

A product obtained by expressing a gene using genetic engineering techniques means a product which is obtained by linking a ligated gene composed of a gene encoding the antigen of interest and a gene encoding a substance which binds to B cells (this ligated gene is obtainable either in nature or by synthesis) to an expression vector, expressing the ligated gene in a host microorganism in large quantity, and then purifying the expressed product. For example, a protein which is obtained by introducing a gene encoding a substance which binds to B cells into the upstream of a gene encoding an antigen, and expressing the resultant gene may be given. Such a product is prepared by the method described below.

Briefly, a ligated gene composed of a gene encoding an antigen and a gene encoding a substance which binds to B cells (e.g., a gene encoding an antibody to a B cell surface antigen) (both genes can be obtained in nature or by synthesis by conventional methods) is inserted into an expression vector to thereby construct an expression plasmid. Subsequently, all or a part of the DNA comprising the ligated gene described above is cut out with appropriate restriction enzymes. The DNA thus obtained is linked to the downstream of an appropriate promoter and then introduced into a host which is capable of transforming. Thus, the host is transformed. As the vector DNA used here, plasmid vector, virus vector and the like may be enumerated. As the host, yeast, *E. coli*, animal cells, insect cells, and the like may be enumerated. The transformant obtained above is cultured in a conventional medium. Then, the culture (culture solution, cultured microorganisms, cultured cells or culture supernatant) is collected and purified by conventional methods.

A culture supernatant after cultivation may be obtained by centrifuging the culture solution to remove transformed cells. Alternatively, a culture supernatant may be obtained by harvesting transformed cells, suspending them in a buffer, for example, freezing them, and subjecting them to thawing treatment, boiling treatment, etc. and then centrifuging them.

In order to separate and purify the conjugate of an antigen and a substance which binds to B cells (a recombinant type conjugate) from the culture obtained, conventional methods for purifying a protein may be used. For example, the purification may be performed by a suitable combination of salting out, centrifugation, various chromatographies, electrophoresis and the like.

As various chromatographies, gel filtration, ion exchange chromatography, reversed-phase chromatography, affinity chromatography and the like may be enumerated. The confirmation of the purity and the molecular weight of a purified product is performed by SDS (sodium lauryl sulfate) polyacrylamide gel electrophoresis, Western blotting and the like.

4) To effect immunosuppression against cell- or tissue-derived antigens such as histocompatibility antigen is useful in treating or preventing, for example, rejection reactions at the time of organ transplantation and graft-versus-host diseases (GVHD) at the time of bone marrow transplantation. To effect immunosuppression against allergy causative substances (such as the pollen of Japanese cedar) is useful in treating or preventing, for example, allergic rhinitis caused by pollen. To effect immunosuppression against food protein antigens (such as ovalbumin) is useful in treating or preventing food allergy. Also, to effect immunosuppression against artificial antigens (such as drugs) is useful in treating or preventing drug allergy, drug-induced autoimmune diseases and the like.

(3) Contact Treatment of B Cells with an Antigen

The immunosuppressant of the invention can be obtained by contact-treating B cells with an antigen. In the present invention, the contact treatment of B cells with an antigen means, for example, (i) culturing B cells and an antigen in an appropriate medium and then recovering the B cells; (ii) cell fusion method, e.g., contacting a liposome encapsulating or attaching to itself an antigen (an antigen-containing liposome) with B cells to allow cell fusion and then recovering the fused cells or (iii) injecting an antigen into B cells by microinjection and recovering the B cells into which the antigen has been injected. When B cells are treated as described in (i) or (ii) above, the antigen once attaches to the surface of B cells and then it is taken into the cells, or the antigen is directly taken into the B cells. Anyway, it is considered that the antigen is eventually taken into the cells.

Of these contact treatments, when B cells are contact-treated with an antigen *per se*, or when B cells are contact-treated with a conjugate of antigen/substance binding to B cells, these treatments may be performed by conventional cell culture methods. When B cells are contact-treated with a liposome encapsulating or attaching to itself an antigen (an antigen-containing liposome), the treatment may be performed by the conventional cell fusion method.

Hereinbelow, specific techniques will be described.

1) Method by Culturing

The B cells as prepared in (1) above are cultured with the antigen described in (2) above to thereby contact the B cells with the antigen. Thus, the antigen attaches to or is taken into the B cells.

Cultivation is performed as follows.

The B cells are suspended in a medium (e.g., RPMI-1640 medium containing 10% FCS). The antigen is added thereto and the cells are cultured therein. The mixing ratio between the antigen and the B cells is not particularly limited and may be appropriately selected. The time of culturing (i.e., the time for sensitizing the B cells with the antigen) is from 90 minutes to 18 hours. This time may be appropriately adjusted depending on the kind and characters of the antigen used. The culturing may be performed under conventional conditions. For example, it may be performed at a $CO_2$ concentration of 5% and at 37°C. Specifically, for example, a cell suspension at a density of $1x10^7$ cells/4 ml (RPMI-1640 medium containing 10% FCS) is prepared. To this suspension, ovalbumin (OVA) (the antigen) is added at a rate of 50 μg/ml and cultured for 90 minutes (5% $CO_2$, 37°C).

After culturing for the specific period, the culture solution containing the B cells and the antigen is washed to remove the antigen. For the washing, the culture solution is centrifuged at 300-400xG for 5-10 minutes and the supernatant is removed. The remainder is re-suspended in antigen-free MEM medium or the like.

By such treatment, the antigen-treated B cells of the invention are obtained through the attachment of the antigen to the B cells and the subsequent incorporation into the cells or in a different manner.

2) Method by Cell Fusion

As an example of a method by cell fusion, contact treatment using a liposome may be given. Briefly, the B cells as prepared in (1) above are fused to a liposome obtained by attaching thereto the antigen described in (2) above or by allowing the antigen to be encapsulated therein. Thus, the antigen is allowed to be attached to or taken into the B cells. Alternatively, a method in which a liposome encapsulating or attaching to itself the antigen or red blood cells are fused to the B cells, or a method in which antigen-antibody reaction is used may be enumerated.

For example, the antigen is encapsulated in or attached to a liposome by the Ca-EDTA method. Then, the resultant liposome is fused to the B cells by glycerol treatment. Briefly, 5 μM phosphatidylserine and 5 μM cholesterol are suspended in 1 ml of PBS and subjected to sonication for 60 minutes. $CaCl_2$ is added to give a final concentration of 2 mM and the suspension is kept at 37 °C for 60 minutes. The cylinder (white precipitate) generated is collected by centrifugation (2500xG, 10 minutes). A solution containing the antigen (0.5 ml) is added thereto and agitated. 15 mM EDTA-Na (pH is adjusted to 7.4 with NaOH) is added thereto and agitated. By keeping the resultant solution at 37 °C for 30 minutes, a liposome is formed. This liposome is washed twice with PBS (48,000xG, 20 minutes) and suspended in PBS or the like. To the B cells suspended in MEM medium and kept at 37 °C , the resultant liposome suspension is added directly and kept at 37°C for 30 minutes. The medium is adjusted so that it contains 25% glycerol and treated for 4 minutes. Thereafter, the cells are washed with MEM medium twice and transferred to a fresh medium.

Thus, the antigen-containing liposome is prepared through the encapsulation of the antigen into the liposome or attachment of the antigen on the liposome.

3) Method by Microinjection

A method by microinjection is a means employed for allowing an antigen to be incorporated into B cells. In other words, an antigen is directly introduced into B cells by the conventional method.

(4) Immunosuppression Method

Immunosuppression of a human or animal is effected by transferring the B cells obtained by the treatment described above (antigen-treated B lymphocytes) into the human or animal.

The term "transferring" means injecting the B cells which were obtained by culturing B cells with an antigen and then removing the antigen by washing (i.e., antigen-treated B lymphocytes) through veins by intravenous injection, intravenous drip, etc., or transplanting the B cells directly into tissues, or the like. Transfer is not limited to only one time, but several times of transfer, i.e., re-transfer is also included. In the present invention, "transplantation" and "transfer" may be used in the same meaning.

The "human or animal" may or may not be the same human or animal from which B cells were collected in (1) above; the "human or animal" may or may not be autologous, isologous or allogeneic to the above human or animal.

The thus immunosuppressed human or animal exhibits an immunosuppressive effect only against the antigen used in (3) above. Therefore, immune functions other than the one which was immunosuppressed are not suppressed. Thus, unlike conventional immunosuppressants which contribute to the treatment of allergy but cause infections, the immunosuppressant of the present invention will not cause such inconvenience. For example, when the immunosuppressant of the invention comprises B cells obtained by culturing with pollen, this immunosuppressant only suppresses the immune response to pollen, thus being effective for preventing and treating the so-called pollinosis. On the other hand, since this immunosuppressant does not suppress other immune functions, it can prevent complications such as infections.

The confirmation as to whether immunosuppression has been effected or not can be made by determining antibody production or the function of lymphocytes such as T cells. The determination of the amount of antibody may be performed by conventional methods such as ELISA. The determination of T cell functions may be performed by conventional methods. For example, the proliferation ability of T cells is determined by isotope intake method, or the cytokine production from T cells is determined with a special kit for this purpose or by bioassay.

(5) Immunosuppressant

The immunosuppressant of the invention is effective mainly for treating or preventing rejection reactions or GVHD after organ transplantation; allergy; or the immune diseases listed up in Table 1 above.

As a method for administering the immunosuppressant of the invention, parenteral administration may be given. Parenteral administration includes injections such as intravenous injection, intravenous drip, and intratissular injection. The amount of dose varies depending on the age of the subject, the route of administration and the times of administration, and may vary in a wide range. The effective dose (effective B cell count) to be administered as a combination of an effective amount of the immunosuppressant of the invention, an appropriate diluent and a pharmaceutically acceptable carrier is $10\text{-}10^{10}$ cells/kg body weight/day. This is administered one time a day or divided into several times. The administration is carried out for 1 day or more.

When the immunosuppressant of the invention is administered parenterally, the immunosuppressant contains additives such as a stabilizer, buffer, preservative, isotonicity inducing agent, etc. and is prepared at the time of use.

<u>Brief Description of the Drawings</u>

Fig. 1 is a graph showing a decrease in the production of anti-OVA antibody.

Fig. 2 is a graph showing a decrease in the production of anti-OVA antibody.

Best Mode for Carrying Out the Invention

Hereinbelow, the present invention will be described more specifically with reference to the following Example. However, the present invention is not limited to this Example.

[Example 1] Preparation of B Cells

Spleens of 5 to 11 week-old C57/BL6 female mice were removed and placed in plastic laboratory dishes containing a culture solution (MEM medium). By applying pressure to the spleen, intrasplenic lymphocytes were released therefrom. After lightly suspended with a pipette, the lymphocytes were passed through a metal mesh and then centrifuged at about 100xG. The precipitate was removed to thereby prepare a suspension of cells made into single cells. The cells were washed 2-3 times repeatedly by centrifuging the suspension at about 300xG and removing the supernatant. Finally, the cells in the form of single cell were suspended in RPMI-1640 containing 1% BSA. To this cell suspension, anti-Thy-1 antibody (ICN immunoBiologicals) was added and reacted at 4°C for 30 minutes. Then, a complement (Cedarlane) was added thereto and the cells were cultured at 37°C for 30-40 minutes to remove T lymphocytes. Subsequently, the resultant cell solution was applied to a Sephadex G-10 column (Pharmacia) twice. Cells adhering to the column were removed and non-adhesive cells eluting from the column were obtained as B cells.

[Test Example 1]

Hereinbelow, Test Examples of the immunosuppressant of the invention will be described to explain the pharmacological effect (immunosuppressive action) of the immunosuppressant.

The B cells obtained in Example 1 above (approx. $1x10^7$ cells/mouse) were washed by centrifugation and then suspended in RPMI-1640 medium containing 10% FCS. To this suspension, OVA which is an antigen was added to give a final concentration of 50 μg/ml and the cells were cultured at 37°C for 90 minutes. Then, the suspension was centrifuged at 300-400xG for 5-10 minutes and the supernatant was removed. Thereafter, a fresh medium (MEM) was added to re-suspend the cells and the resultant suspension was centrifuged again. This centrifugation was repeated 2-3 times for washing. Through these washing operations, OVA was removed.

The resultant B cells ($3x10^6$ cells) were suspended in MEM medium and intravenously transplanted to mice. Four days and about 18 days after the transplantation, primary sensitization and secondary sensitization were performed by intravenous administration of 200 μg and 100 μg of the same antigen, respectively. One to two weeks after each sensitization, the amount of anti-OVA antibody in blood was determined using the absorbance at a wave length of 492 nm ($A_{492}$) as an indicator. The antibody production suppression ratio was calculated by the following formula.

$$\text{Antibody Production Suppression Ratio (\%)} = [(A_{492} \text{ in Positive Control Group} - A_{492} \text{ in Test Group}) / (A_{492} \text{ in Positive Control Group} - A_{492} \text{ in Negative Control Group})] \times 100$$

In the positive control group, the antigen-sensitized B cells (i.e., B cells contact-treated with the antigen) were not transplanted, but a serum dilution from a mouse immunized with OVA (soluble) alone was used. In the negative control group, a serum dilution from a non-treated mouse was used.

As a result, in both of the primary and the secondary immune reaction, while a very high anti-OVA antibody production was observed in positive control mice which were sensitized with the antigen without transplantation of B cells, anti-OVA antibody production was suppressed in those mice into which the antigen-treated B cells (i.e., the immunosuppressant of the invention) were transplanted. In particular, a suppression ratio of about 95% was recognized in the secondary immune reaction. The results are shown in Table 2.

Table 2

| | Anti-OVA antibody production [a] | |
|---|---|---|
| | Primary immune reaction [b] | Secondary immune reaction [c] |
| No treatment | 0.303 | 0.176 |
| Treatment with OVA alone | 1.945±0.668 | 2.643 ±0.060 |
| Treated with B cells and OVA | 0.708±0.191 (43.1%) [d] | 0.316 ±0.061 (94.4%) [d] |

a) : Absorbance at a wave length of 492 mm by ELISA
b) : Serum dilution 1/50
c) : Serum dilution 1/250
d) : Antibody production suppression ratio

[Test Example 2]

The B cells obtained in Example 1 above were washed by centrifugation and then suspended in RPMI-1640 medium containing 10% FCS. To this suspension, OVA (different two lots (Nos. P89301 and P91501) were used; manufactured by Biochemical Industries), an antigen, was added to give a final concentration of 50 μg/ml and the cells were cultured at 37°C for about 18 hours. Then, the suspension was centrifuged at 300-400xG for 5-10 minutes and the supernatant was removed. Thereafter, a fresh medium (MEM) was added to re-suspend the cells and the resultant suspension was centrifuged again. This centrifugation was repeated 2-3 times for washing. Through these washing operations, OVA was removed.

The resultant B cells ($6x10^5$ cells) were suspended in MEM medium and intravenously transplanted to mice. Four days and about 19 days after the transplantation, primary sensitization and secondary sensitization were performed by intravenous administration of 200 μg and 100 μg of the same antigen, respectively. One to two weeks after each sensitization, the amount of anti-OVA antibody in blood was determined using the absorbance at a wave length of 492 nm ($A_{492}$) as an indicator.

The positive control group and the negative control group were treated in the same manner as described above.

The results are shown in Fig. 1.

In both of the primary and the secondary immune reaction, a high anti-OVA antibody production was observed in positive control mice which were sensitized with the antigen without transplantation of B cells (see #7-#9 in Fig. 1). On the other hand, with respect to those mice into which the antigen-treated B cells (i.e., the immunosuppressant of the invention) were transplanted, anti-OVA antibody production was suppressed in half of them (3 out of 6) (see #4-#6 in Fig. 1). In mice #1-#3 and mice #4-#6, the B cells transplanted to them had been treated with OVA of different lot numbers (lot Nos. P89301 and P91501, respectively). Mouse #10 is a negative control (which was administered a serum dilution from a non-treated mouse).

[Test Example 3]

OVA (egg albumin, 5 x crystalized; Biochemical Industries; lot No. P89301) was dissolved in PBS(-) at a low temperature to give a concentration of 1 mg/ml. This solution was filtration-sterilized through a Millipore filter 0.22 μm in pore size. Then, OVA polymers were prepared by incubating the solution at 70°C for 18 hours while keeping the solution stationary. Although, under these conditions, the co-existence of non-polymerized OVA (molecules not changed in molecular weight) was confirmed by analysis using SDS-PAGE, no separation operation was performed particularly. The resultant polymers were diluted with a medium, added to a B cell suspension to give a final concentration of 50μg/ml, and incubated *in vitro* at 37°C for about 90 minutes to thereby perform pulse labeling. Then, the suspension was centrifuged at 300-400xG for 5-10 minutes and the supernatant was removed. Thereafter, a fresh medium (MEM) was added to re-suspend the cells and the resultant suspension was centrifuged again. This centrifugation was repeated 2-3 times for washing. Through these washing operations, OVA was removed.

Subsequently, the same procedures as described in Test Example 2 were performed, and the amount of antibody was determined using the absorbance at a wave length of 492 nm ($A_{492}$) as an indicator. The positive control group and the negative control group were treated in the same manner as described above.

The results are shown in Fig. 2. From Fig. 2, it is shown that even B cells treated with thermally denatured antigen (OVA polymer; mark "□" in Fig. 2) suppress the antibody production compared to untreated B cells (mark "◊" in Fig. 2).

Industrial Applicability

According to the present invention, an immunosuppressant is provided which suppresses immune reaction with a specific antigen. The immunosuppressant of the invention is useful for treatment to suppress rejection reaction in organ transplantation and graft-versus-host diseases (GVHD) in bone marrow transplantation, as well as for prevention of allergy.

**Claims**

**1.** An immunosuppressant comprising antigen-treated B lymphocytes obtained by contact-treating B lymphocytes with an antigen.

**2.** An immunosuppressant comprising antigen-treated B lymphocytes obtained by contact-treating B lymphocytes with an antigen, a liposome encapsulating or attaching to itself an antigen, or a conjugate of an antigen and a substance which binds to B lymphocytes, or a mixture of at least two of these materials.

**3.** The immunosuppressant of claim 1, wherein the antigen is a cell- or tissue-derived antigen, a causative antigen of allergy, or a causative antigen of an autoimmune disease.

**4.** The immunosuppressant of claim 2, wherein the antigen is a cell- or tissue-derived antigen, a causative antigen of allergy, or a causative antigen of an autoimmune disease.

**5.** The immunosuppressant of claim 2 or 4, wherein the substance which binds to B lymphocytes is an antibody, a part thereof, or a crosslinking agent.

**6.** The immunosuppressant of claim 2 or 4, wherein the conjugate of an antigen and a substance which binds to B lymphocytes is a conjugate that is obtained by expressing a gene comprising a gene encoding the antigen and a gene encoding the substance which binds to B lymphocytes.

**7.** The immunosuppressant of claim 6, wherein the substance which binds to B lymphocytes is an antibody or a part thereof.

**8.** A method for creating an immunosuppressed animal comprising transferring to an animal the immunosuppressant of any one of claims 1 to 7.

**9.** The method of claim 8, wherein the animal is an experimental animal.

**10.** An immunosuppressed animal created by the method of claim 8 or 9.

**11.** A method for treating a disease comprising administering to a human the immunosuppressant of any one of claims 1 to 7.

**12.** A method for preventing a disease comprising administering to a human the immunosuppressant of any one of claims 1 to 7.

**13.** The method of claim 11, wherein the disease is graft rejection, allergy, an autoimmune disease or a graft-versus-host disease.

**14.** The method of claim 12, wherein the disease is graft rejection, allergy, an autoimmune disease or a graft-versus-host disease.

**15.** An antigen-containing liposome which is encapsulating or attaching to itself the antigen.

**16.** The liposome of claim 15, wherein the antigen is a cell- or tissue-derived antigen, a causative antigen of allergy, or a causative antigen of an autoimmune disease.

**17.** A conjugate of an antigen and a substance which binds to B lymphocytes.

18. The conjugate of claim 17, wherein the antigen is a cell- or tissue-derived antigen, a causative antigen of allergy, or a causative antigen of an autoimmune disease.

19. The conjugate of claim 17, wherein the substance which binds to B lymphocytes is an antibody, a part thereof or a crosslinking agent.

20. A conjugate of an antigen and a substance which binds to B lymphocytes, obtained by expressing a gene comprising a gene encoding the antigen and a gene encoding the substance which binds to B lymphocytes.

21. The conjugate of claim 20, wherein the antigen is a cell- or tissue-derived antigen, a causative antigen of allergy, or a causative antigen of an autoimmune disease.

22. The conjugate of claim 20 or 21, wherein the substance which binds to B lymphocytes is an antibody or a part thereof.

23. A composition comprising the liposome of claim 15 or 16 and/or the conjugate of any one of claims 17 to 22.

24. An antigen which is able to induce an immunosuppressive action in B lymphocytes when contact-treated with the B lymphocytes.

25. The antigen of claim 24, wherein the B lymphocytes have been collected from an animal body.

26. The immunosuppressant of any one of claims 1 to 7, which is used to immunosuppress an animal by collecting B lympocytes from the animal body, inducing an immunosuppressive action in the lymphocytes and transferring the lymphocytes to the animal body

FIG. 1

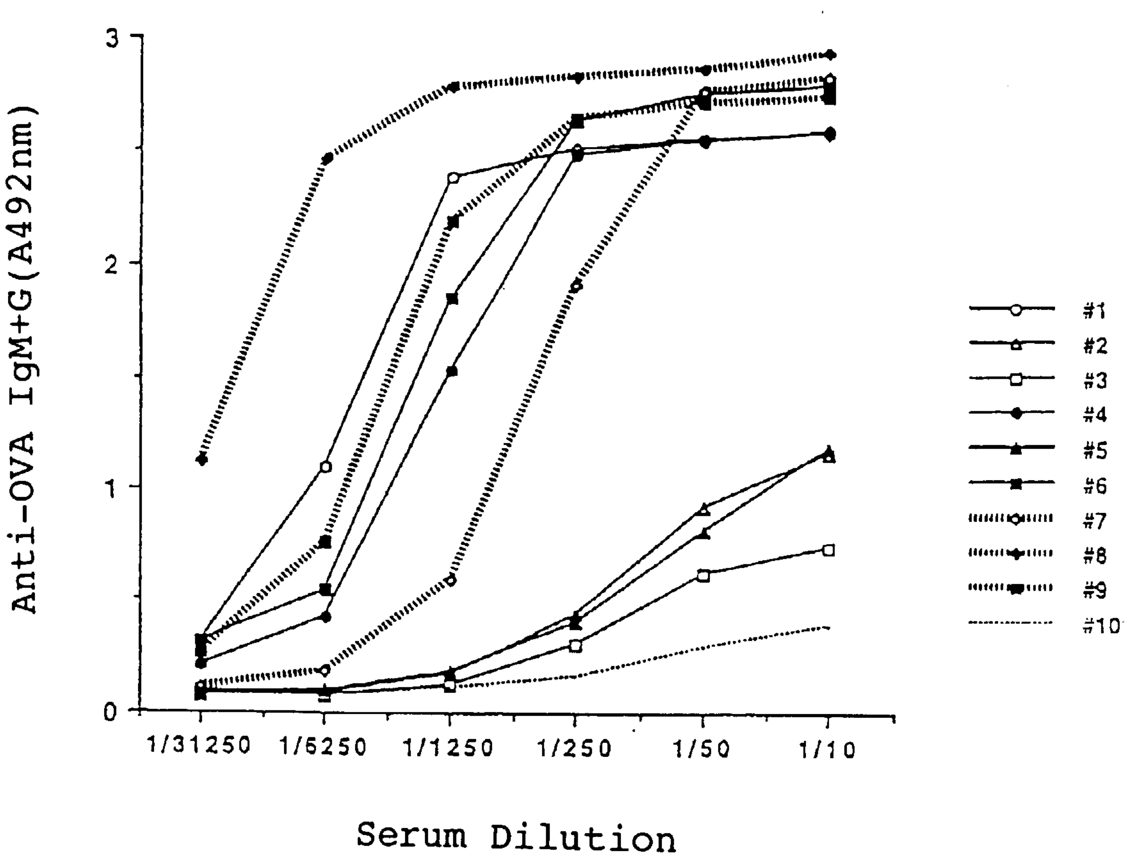
3
2
1
0
Anti-OVA IgM+G(A492nm)
1/31250
1/6250
1/1250
1/250
1/50
1/10
Serum Dilution
#1
#2
#3
#4
#5
#6
#7
#8
#9
#10

FIG.2

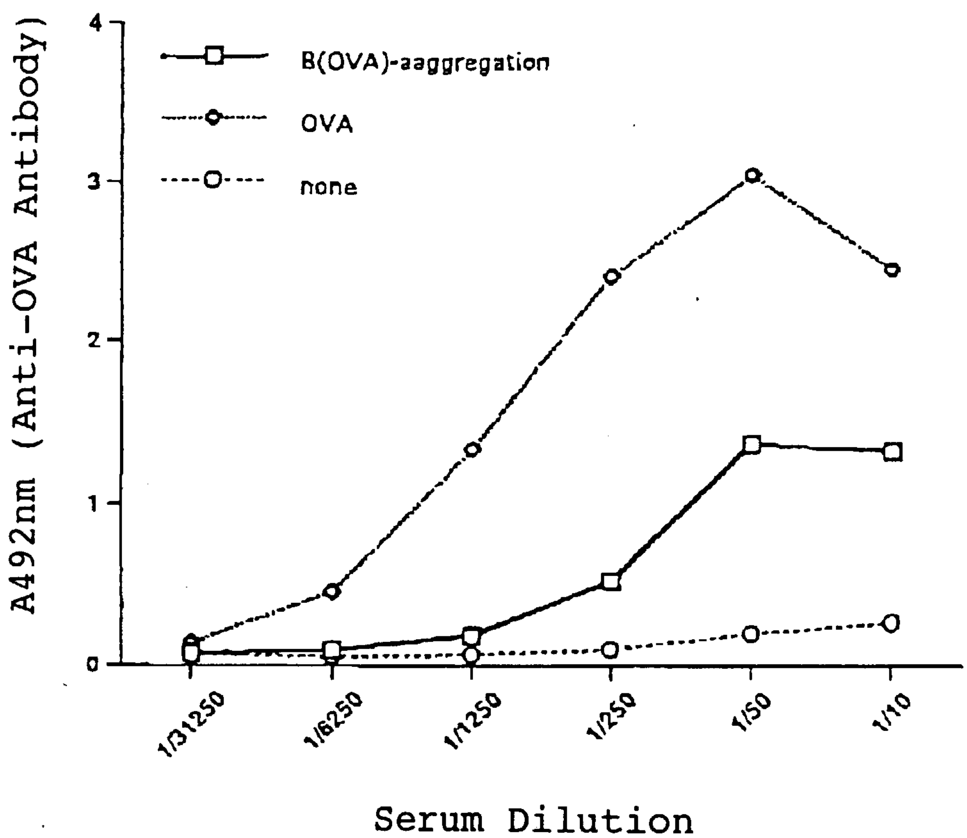
A492nm (Anti-OVA Antibody)
4
3
2
1
0
B(OVA)-aaggregation
OVA
none
1/31250
1/6250
1/1250
1/250
1/50
1/10
Serum Dilution

## INTERNATIONAL SEARCH REPORT

International application No.
PCT/JP96/00959

**A. CLASSIFICATION OF SUBJECT MATTER**

Int. $Cl^6$ A61K35/14, A61K39/00

According to International Patent Classification (IPC) or to both national classification and IPC

**B. FIELDS SEARCHED**

Minimum documentation searched (classification system followed by classification symbols)

Int. $Cl^6$ A61K35/14, A61K39/00

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| T | Chemical Abstracts, Abstract No. 124:211609 (1996) (Abstract of Shek, P.N.; Sabiston, B.H. "Lipisome-mediated immune response:cellular requirements and immunomodulation by bifunctional liposomes." Drug Targeting Delivery, Volume Date 1995, 6, 19-34(1995)) | 1 - 26 |
| A | Chemical Abstracts, Abstract No. 84:119824 (1976) (Abstract of Diamantstein, T.; Keppler, W.; Blitstein-Willinger, Eveline; Ben-Efraim, S. "Suppression of the primary immune response in vivo to sheep red blood cells by B-cell mitogens." Immunology, 30(3), 401-407(1976)) | 1 - 26 |
| A | JP, 58-96029, A (Nippon Kotai Kenkyusho K.K.), June 7, 1983 (07. 06. 83)(Family: none) | 1 - 26 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| June 10, 1996 (10. 06. 96) | June 18, 1996 (18. 06. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)